# EUROPÄISCHE PATENTSCHRIFT

(11) **EP 3 074 083 B1**
(45) Veröffentlichungstag und Bekanntmachung des Hinweises auf die Patenterteilung: **19.10.2022**
(21) Anmeldenummer: 14815260.6
(22) Anmeldetag: 27.11.2014
(51) Int. Cl.: A61N 1/05

(54) **VORRICHTUNG ZUR TRANSKUTANEN IMPLANTATION EPIKARDIALER HERZSCHRITTMACHERELEKTRODEN**
DEVICE FOR THE TRANSCUTANEOUS IMPLANTATION OF EPICARDIAL PACEMAKER ELECTRODES
DISPOSITIF D'IMPLANTATION TRANSCUTANÉE D'ÉLECTRODES ÉPICARDIQUES DE STIMULATEUR CARDIAQUE

(30) Priorität: 27.11.2013 DE 102013224283
(43) Veröffentlichungstag der Anmeldung: 05.10.2016
(73) Patentinhaber: Deutsches Herzzentrum Berlin, 13353 Berlin (DE)
(72) Erfinder: BARTOSCH, Marco, 10829 Berlin (DE); PETERS, Heiner, 28203 Bremen (DE); SCHMITT, Boris, 10435 Berlin (DE); PETERS, Björn, 10437 Berlin (DE)
(74) Vertreter: Maikowski & Ninnemann Patentanwälte Partnerschaft mbB
(86) Internationale Anmeldenummer: PCT/EP2014/075821
(87) Internationale Veröffentlichungsnummer: WO 2015/078971

(56) Entgegenhaltungen:
- EP-A1- 1 661 600
- WO-A2-2006/116310
- US-A1- 2005 182 465
- US-A1- 2010 087 725
- US-B1- 7 369 901
- ANDRE D'AVILA ET AL: "Pericardial Anatomy for the Interventional Electrophysiologist", JOURNAL OF CARDIOVASCULAR ELECTROPHYSIOLOGY, Bd. 14, Nr. 4, 1. April 2003 (2003-04-01), Seiten 422-430, XP055174312, ISSN: 1045-3873, DOI: 10.1046/j.1540-8167.2003.02487.x

## Beschreibung

Die Erfindung betrifft eine Vorrichtung zur transkutanen Implantation einer epikardialen Herzschrittmacherelektrode, die in einem schlauchförmigen, flexiblen und in den Perikardspalt einführbaren Implantationskatheter angeordnet ist.

Ein derartiger Implantationskatheter wird nach einer Punktion des Herzbeutels (Perikards) in den zwischen dem Epikard und der Lamina parietalis des Perikards gebildeten schmalen, abgeschlossenen Hohlraum, der Perikardhöhle oder dem Perikardspalt, eingeführt und bis zum Implantationsort vorgebracht. Am Implantationsort wird die epikardiale Herzschrittmacherelektrode, die aus einer elektrisch isolierten Elektrodenleitung mit einem oder mehreren nicht isolierten distalen Kontaktunkten besteht, mittels eines aktiven Fixierungsmechanismus (z.B. einer Helix) im Epikard-Myokard verankert. Über die so fixierte Elektrode können die von einem an das proximale Ende der Elektrode angeschlossenen Herzschrittmacher abgegebenen elektrischen Impulse auf das Myokard übertragen werden und es wird somit eine kontrollierte Stimulation des Herzmuskels ausgelöst.

Wegen der hierfür erforderlichen Punktion des Perikards und des sehr schmalen Perikardspaltes soll der Implantationskatheter einen möglichst geringen Durchmesser aufweisen, um einen minimalinvasiven Eingriff zu gewährleisten, die Gefahr einer Verletzung von umgebenden Organen oder Organteilen (z.B. Pleura, Zwerchfell, Myokard oder Herzkranzgefäßen) zu minimieren und eine ausreichende räumliche Manipulation zu ermöglichen.

Aus der EP 2 266 657 A1 ist eine implantierbare Elektrodensonde mit einem schlauchartigen, flexiblen Sondenkörper, einer am distalen Ende des Sondenkörpers angeordneten Elektrode, sowie einer im Sondenkörper geführten elektrischen Zuleitung zur Elektrode bekannt, bei der ein mit dem distalen Endabschnitt in Wirkverbindung stehender Bewegungsmechanismus von einem ersten Zustand in einen in Bezug auf den ersten Zustand radial verbreiterten zweiten Zustand überführbar ist, in der er in Bezug auf die radiale Abmessung des übrigen Sondenkörpers und der am distalen Ende des Sondenkörpers angeordneten Elektrode radial verbreitert ist. Dadurch kann die Elektrodensonde mit einem dünnen distalen Endabschnitt durch eine Einführschleuse und Gefäße implantiert werden, während am Implantationsort der distale Endabschnitt des Sondenkörpers radial verbreitert werden kann, um einerseits einen ausreichenden Perforationsschutz zum Verhindern einer Gewebeperforation durch die Elektrode und andererseits eine große Fläche für die lokale Abgabe eines Wirkstoffs am Implantationsort zur Verfügung zu stellen.

Zur sicheren Verankerung von Herzschrittmacherelektroden im Epikard sind spiral- oder korkenzieherförmige Elektroden bekannt, die in das Epikard/Myokard mittels einer Vorrichtung zum Drehen und Einführen einer Epikard/-Myokard-Elektrode eingeschraubt werden. Dabei wird die Elektrode mit der Mittelachse der Spiralelektrode senkrecht zur Oberfläche des Myokardgewebes implantiert, was gemäß der DE 60 2004 005 845 T2 mittels einer Vorrichtung zum Drehen und Einführen einer Epikard/Myokard-Herzelektrodenleitung erfolgt, die eine Spiralelektrode, einen Elektrodenkopf, einen Elektrodenkörper, einen länglichen Schaft und eine drehbar über dem länglichen Schaft angeordnete drehbare Röhre sowie ein distales Element enthält, das den Elektrodenkopf umfasst und schwenkbar mit einem distalen Abschnitt des länglichen Schaftes und mit der drehbaren Röhre gekoppelt ist, so dass der Elektrodenkopf durch Verschwenken des distalen Elements in einem vorgebbaren Winkel in das Myokardgewebe eingeschraubt werden kann.

Aufgrund des sehr engen Perikardspaltes ist der Einsatz einer derartigen, Platz beanspruchenden Vorrichtung zum Einschrauben einer epikardialen Herzschrittmacherelektrode ungeeignet. In der US 2005/182465 A1 werden weitere Vorrichtungen beschrieben, die einen Zugang zum Perikardspalt ermöglichen.

Aufgabe der vorliegenden Erfindung ist es, eine Vorrichtung zur transkutanen bzw. transthorakalen Implantation einer epikardialen Herzschrittmacherelektrode mittels eines in den Perikardspalt einführbaren Implantationskatheters bereitzustellen, mit der eine Implantation der epikardialen Herzschrittmacherelektrode in das Epikard/Myokard in einem vorgebbaren, geeigneten Implantationswinkel reproduzierbar und in stabiler Lage ermöglicht wird. Diese Aufgabe wird erfindungsgemäß mit den Merkmalen des Anspruchs 1 gelöst.

Die erfindungsgemäße Lösung, eine Vorrichtung zur transkutanen Implantation einer mit einem distalen Fixierungsmechanismus versehenen epikardialen Herzschrittmacherelektrode mittels eines in den Perikardspalt einführbaren Implantationskatheters bereitzustellen, bei dem der distale Endbereich der Elektrode mit einem formveränderlichen Element zur Ausrichtung und insbesondere zur Einstellung des Implantationswinkels und zur seitlichen Stabilisierung der Elektrode verbunden ist, ermöglicht und gewährleistet eine reproduzierbare und stabile Implantation der Elektrode in einem vorgebbaren, geeigneten Winkel in das Epikard/Myokard.

Die Führung des Elektrodenendes inklusive des distalen Fixierungsmechanismus mittels des formveränderlichen Elementes gewährleistet einerseits, dass die Elektrode bei der transkutanen Einführung des Implantationskatheters in den Perikardspalt koaxial zum Implantationskatheter ausgerichtet ist und somit ein minimalinvasives Eindringen in das Körpergewebe eines Patienten ermöglicht, andererseits wird ein sicheres Einbringen der epikardialen Herzschrittmacherelektrode in das Epikard/Myokard durch das formveränderliche Element ermöglicht, indem die Elektrodenspitze mit dem Fixierungsmechanismus durch eine Form- und ggf. zusätzlich eine Volumenveränderung des formveränderlichen Elements in einem definierten Winkel zur Oberfläche des Epikards ausgerichtet wird und in dieser Ausrichtung stabilisiert werden kann, so dass eine sichere Verankerung der Elektrode mit einem definierten Eindringwinkel in das Epikard/Myokard gewährleistet ist.

Unter der Bezeichnung Elektrode bzw. Herzschrittmacherelektrode wird ein schlauchförmiger, biegsamer Sondenkörper mit einem in einer Isolationshülle angeordneten Innenleiter, der beispielsweise in Form einer Drahtwendel ausgebildet ist, verstanden. Der Sondenkörper enthält einen distalen Endabschnitt mit einer Elektrodenspitze und einem Fixierungsmechanismus und einen proximalen Endabschnitt mit einem Anschluss, beispielsweise in Form eines Steckers, der mit einem Herzschrittmacher verbunden wird.

Das formveränderliche Element zur Einstellung des Implantationswinkel und seitlichen Stabilisierung der epikardialen Herzschrittmacherelektrode besteht in einer ersten, nicht beanspruchten Ausführungsform aus einem befüllbaren, d.h. aufblasbaren oder auffüllbaren Ballon, der über eine Gas- oder Flüssigkeitszuleitung mit dem proximalen Ende des Implantationskatheters verbunden ist.

In dieser ersten Ausführungsform wird die leere Ballonhülle im distalen Endbereich des Implantationskatheters angeordnet, wo die Ballonhülle ein minimales Volumen einnimmt, so dass auch der Implantationskatheter mit einem minimalen Durchmesser ausgebildet werden kann. Am Implantationsort wird die Ballonhülle beispielsweise durch Zurückziehen des Außenkatheters freigegeben und kann durch Gas- oder Flüssigkeitszufuhr vom proximalen Ende des Implantationskatheters über die Gas- oder Flüssigkeitsleitung auf ein solches Volumen aufgeblasen oder aufgefüllt werden, dass die entlang der Außen-Kontur des Ballons herumgeführte epikardiale Herzschrittmacherelektrode in einen optimalen Implantationswinkel zum Epikard/Myokard ausgerichtet und gleichzeitig seitlich stabilisiert wird, so dass die epikardiale Herzschrittmacherelektrode sicher in das Epikard/Myokard eingeführt, vorzugsweise eingeschraubt werden kann.

In bevorzugter Ausführungsform ist das distale Ende der Elektrode in einem offenen oder geschlossenen Führungskanal um den Ballon herum geführt.

Die Anordnung der Elektrode in einem offenen oder geschlossenen Führungskanal gewährleistet, dass die Elektrode beim Einführen und insbesondere beim Einschrauben eines spiral- oder korkenzieherförmigen distalen Fixierungsmechanismus in das Epikard/Myokard nicht seitlich ausweichen kann, was die Gefahr beinhalten würde, dass der optimale Implantationswinkel nicht eingehalten wird. Darüber hinaus gewährleistet der Führungskanal das reibungsarme Einschrauben eines spiral- oder korkenzieherförmigen distalen Fixierungsmechanismus der Elektrode in das Epikard/Myokard, so dass eine optimale Übertragung des Drehmoments erfolgt.

Zur seitlichen Stabilisierung weist das formveränderliche Element vom Ballon abstehende, vorzugsweise diametral zueinander angeordnete Lagestabilisatoren auf, die in bevorzugter Ausführungsform als Teil eines kreuz-, hammer-, walzen- oder balkenförmigen Ballons ausgebildet sind.

Die Anordnung seitlich vom Ballon abstehender Lagestabilisatoren ermöglicht einerseits die Unterbringung der Lagestabilisatoren im schmalen Perikardspalt und andererseits durch eine großflächige Anlage der Lagestabilisatoren am Epikard eine gesicherte Positionierung der epikardialen Herzschrittmacherelektrode am Implantationsort zu deren sicherem Einbringen in das Epikard/Myokard.

In einer zweiten Ausführungsform besteht das formveränderliche Element aus einem die Elektrode aufnehmenden Ausricht- und Stabilisationselement mit endseitigen offenen oder geschlossenen Ringen und die endseitigen Ringe verbindenden Streben, die beim Vorschub des Implantationskatheters von der Punktionsstelle zum Implantationsort im Wesentlichen parallel zur Wand des Außenkatheters ausgerichtet sind und sich nach Freisetzen des Ausricht- und Stabilisationselements zu einer Implantationsposition entfalten, wobei in der Implantationsposition mindestens eine mittlere Ausrichtungsstrebe die Elektrode ausrichtet und mindestens zwei vorzugsweise zu beiden Seiten der Ausrichtungsstrebe angeordnete Stützstreben die Ausrichtung und Lage der Elektrode stabilisieren.

In dieser zweiten Ausführungsform des formveränderlichen Elements wird anstelle eines aufblasbaren oder auffüllbaren Ballons ein bezüglich seiner Form veränderliches Ausricht- und Stabilisationselement eingesetzt, das zur Implantation zwangsweise in eine Form gebracht wird, in der er im Wesentlichen koaxial zum Außenkatheter ausgerichtet ist, am Implantationsort beispielsweise durch Zurückziehen des Außenkatheters freigegeben wird und sich in eine vorgegebene gekrümmte Form zurückbildet, die sowohl eine stabile Lage der im flexiblen Käfig geführten Elektrode als auch eine Ausrichtung der Elektrode in einen vorgegebenen Implantationswinkel gewährleistet.

Vorzugsweise bestehen die die endseitigen Ringe verbindenden Streben des Ausricht- und Stabilisationselements in der zweiten Ausführungsform des formveränderlichen Elements aus einem aus einem Formgedächtnismaterial, insbesondere aus Nitinol, einem Federmaterial, insbesondere Federstahl oder Kunststoff, oder einem pseudoelastischen Material, insbesondere aus Nitinol. Alternativ ist auch ein mechanischer Formänderungsmechanismus einsetzbar.

Mit dieser Ausführungsform des Ausricht- und Stabilisationselements wird die Pseudoelastizität einer Formgedächtnislegierung genutzt, bei der der gekrümmte, d.h. von der Zylinderform abweichende Zustand, in dem die innerhalb des Ausricht- und Stabilisationselements geführte Elektrode in den optimalen Implantationswinkel gebracht wird, die selbständig eingenommene Form ist. Durch Hineinziehen des Ausricht- und Stabilisationselements in den Außenkatheter wird das Ausricht- und Stabilisationselement in die zum Außenkatheter koaxiale Form gebracht und nimmt zum Implantieren der Elektrode in das Epikard durch Zurückziehen des Außenkatheters wieder die gekrümmte, ursprüngliche Form ein.

Alternativ hierzu kann auch der temperaturabhängige Formgedächtniseffekt beispielsweise des Nitinols genutzt werden, indem die die Ringe verbindenden Streben des Ausricht- und Stabilisationselement bei Raum- oder Körpertemperatur die gewünschte, gekrümmte Form einnehmen, während sie während des Implantationsvorgangs in eine dem Außenkatheter angepasste Form gebracht werden.

Alternativ können die die endseitigen Ringe verbindenden Streben des Ausricht- und Stabilisationselements aus einem Material mit elastischer Rückstellkraft bestehen, die zur Implantation in eine auf den Außenkatheter ausgerichtete Zwangsform gebracht werden, am Implantationsort eine gekrümmte, dem gewünschten Implantationswinkel angepasste Form nach Freigabe beispielsweise durch Zurückziehen des Außenkatheters einnehmen. Materialien und Formen mit elastischer Rückstellkraft sind beispielsweise Federn, Federstahl oder dergleichen.

Neben Formgedächtnismaterialien und Materialien und Formen mit elastischer Rückstellkraft können auch Polymere oder Materialien eingesetzt werden, die unter Einwirkung eines Magnetfeldes eine gewünschte Form einnehmen, um einerseits eine platzsparende Anordnung des formveränderlichen Elements im Außenkatheter beim Implantieren zu gewährleisten und andererseits am Implantationsort einen optimalen Implantationswinkel bei gleichzeitig lagestabiler Positionierung der epikardialen Herzschrittmacherelektrode zu ermöglichen.

In einer dritten Ausführungsform besteht das formveränderliche Element aus einer Kombination des formveränderlichen Elements in der ersten und zweiten Ausführungsform und weist einen aufblasbaren, über eine Gas- oder Flüssigkeitsleitung mit dem proximalen Ende des Implantationskatheters verbundenen Ballon und ein Ausricht- und Stabilisationselement mit endseitigen Ringen und die endseitigen Ringe verbindenden flexiblen Streben aus einem Formgedächtnismaterial, insbesondere aus Nitinol, auf.

In dieser dritten, kombinierten Ausführungsform dient beispielsweise der Ballon zur Ausrichtung und insbesondere zur Einstellung des Implantationswinkels der Elektrode, während die flexiblen Streben des Ausricht- und Stabilisationselements zur Lagestabilisierung des formveränderlichen Elements am Implantationsort dienen.

Vorzugsweise ist die Elektrode in einem schlauchförmigen, flexiblen, innerhalb des Außenkatheters geführten Innenkatheter angeordnet.

Insbesondere ist das Ausricht- und Stabilisationselement zwischen dem Innenkatheter und dem Außenkatheter angeordnet und mit dem distalen Ende des Innenkatheters verbunden, so dass das während des Implantierens zylinderförmig koaxial zum Außenkatheter ausgerichtete Ausricht- und Stabilisationselement durch Zurückziehen des Außenkatheters seine ursprüngliche oder vorgegebene bzw. einstellbare gekrümmte Form einnimmt.

Wird ein Innenkatheter zur Aufnahme der epikardialen Herzschrittmacherelektrode verwendet, so kann das proximale Ende des Innenkatheters mit einer Vorrichtung zur Anwendung eines Unterdruckes im Innenkatheter verbunden werden. Die Anwendung von Unterdruck im Innenkatheter ermöglicht ein Ansaugen des distalen Endes des Innenkatheters an das Epikard und gewährleistet dadurch eine Fixierung des Innenkatheters am Epikard des Implantationsortes sowie ein sicheres Halten des eingestellten Implantationswinkels oder ermöflicht dessen Vergrößerung. Wird kein Innenkatheter verwendet, kann in Analogie das distale Ende des Außenkatheters an das Epikard angesaugt werden.

Bei der Ausbildung des formveränderlichen Elements in der ersten Ausführungsform als aufblasbarer oder auffüllbarer Ballon kann die zum Ballon führende Gas- oder Flüssigkeitsleitung in der Wand des Innenkatheters, zwischen dem Innenkatheter und dem Außenkatheter oder entlang der Außenseite des Außenkatheters geführt werden.

Um nach der Implantation der epikardialen Herzschrittmacherelektrode im Epikard-Myokard den Innenkatheter und/oder Außenkatheter zu entfernen, ist eine Einrichtung zum längsseitigen Aufschlitzen des Innenkatheters und/oder Außenkatheters beim Herausziehen des Innenkatheters bzw. Außenkatheters aus dem Körpergewebe vorgesehen. Alternativ können die Lumen von Außenkatheter bzw. Außen- und Innenkatheter so groß ausgeführt werden, dass sie von der Elektrode abgezogen werden können.

Dies kann erforderlich sein, wenn ein mit der Elektrode verbundener Stecker am proximalen Ende des Implantationskatheters nach dem derzeit gängigen Industriestandard IS-1 einen größeren Außendurchmesser als die bis zur Elektrodenspitze isodiametrische Elektrode selbst aufweist.

In allen Ausführungsformen kann der Implantationskatheter als steuerbarer Katheter mit einem oder zwei Steuerdrähten am distalen Ende des Implantationskatheters zum Umlenken des Katheters an Biegungen bzw. Krümmungen ausgebildet sein, um einen einfachen Vorschub des Implantationskatheters zur Implantationsstelle zu gewährleisten.

Anhand der in der Zeichnung dargestellten Ausführungsbeispiele soll der der Erfindung zugrunde liegende Gedanke näher erläutert werden. Es zeigen:
- Fig. 1: eine schematische Darstellung eines ein Herz umgebenden Herzbeutels;
- Fig. 2: eine vergrößerte Darstellung des Bereichs II des Herzbeutels gemäß Fig. 1 mit in den Perikardspalt eingeführtem Implantationskatheter und einem Ballon zur Ausrichtung und Lagestabilisierung einer epikardialen Herzschrittmacherelektrode;
- Fig. 3: eine Draufsicht auf den Ballon gemäß Fig. 2 mit seitlich entfaltbaren Flügeln;
- Fig. 4: eine perspektivische Darstellung eines Implantationskatheters mit einem Ballon zur Ausrichtung und Lagestabilisierung einer epikardialen Herzschrittmacherelektrode in der Ausgangsstellung;
- Fig. 5: einen Schnitt durch den Implantationskatheter entlang der Schnittlinie V-V gemäß Fig. 4;
- Fig. 6: eine Draufsicht auf den Implantationskatheter gemäß Fig. 4 ohne Darstellung des Ballons;
- Fig. 7: einen Längsschnitt durch den Implantationskatheter gemäß Fig. 4 ohne Darstellung des Ballons;
- Fig. 8 und 9: verschiedene Ansichten des Implantationskatheters mit aufgeblasenem Ballon zur Ausrichtung und Lagestabilisierung der epikardialen Herzschrittmacherelektrode;
- Fig. 10: eine schematische, perspektivische Darstellung eines in den Außenkatheter eingezogenen Ausricht- und Stabilisationselements zur Ausrichtung und Lagestabilisierung einer epikardialen Herzschrittmacherelektrode;
- Fig. 11 und 12: eine perspektivische Draufsicht und Seitenansicht auf das am Implantationsort entfaltete formveränderliche Element zur Ausrichtung und Lagestabilisierung der epikardialen Herzschrittmacherelektrode gemäß Fig. 10 und
- Fig. 13 und 14: eine perspektivische Ansicht und eine Seitenansicht eines Implantationskatheters mit einer Kombination aus einem aufblasbaren Ballon und einem entfaltbaren Ausricht- und Stabilisationselement zur Ausrichtung und Lagestabilisierung einer epikardialen Herzschrittmacherelektrode.

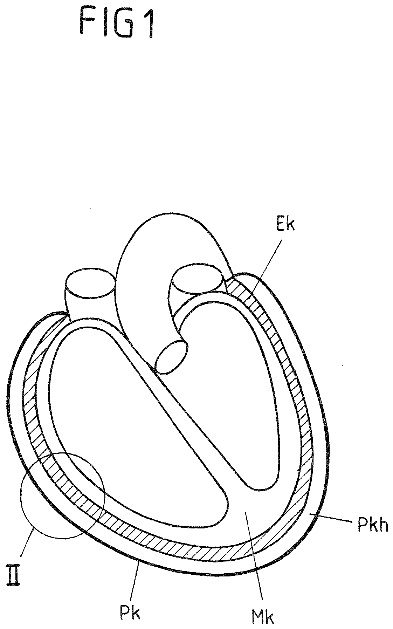

Fig. 1 zeigt in einer schematischen Schnittdarstellung einen doppelwandigen Herzbeutel mit einem das äußere Blatt des Herzbeutels bildenden Perikard Pk, einem das innere Blatt des Herzbeutels bildenden Epikard Ek, welches den Herzmuskel - das Myokard Mk - auskleidet und einem zwischen Epikard Ek und Perikard Pk gebildeten feinen Spalt, die Perikardhöhle oder der Perikardspalt Pkh, die bzw. der mit einer als Gleitfilm dienenden Flüssigkeit gefüllt ist.

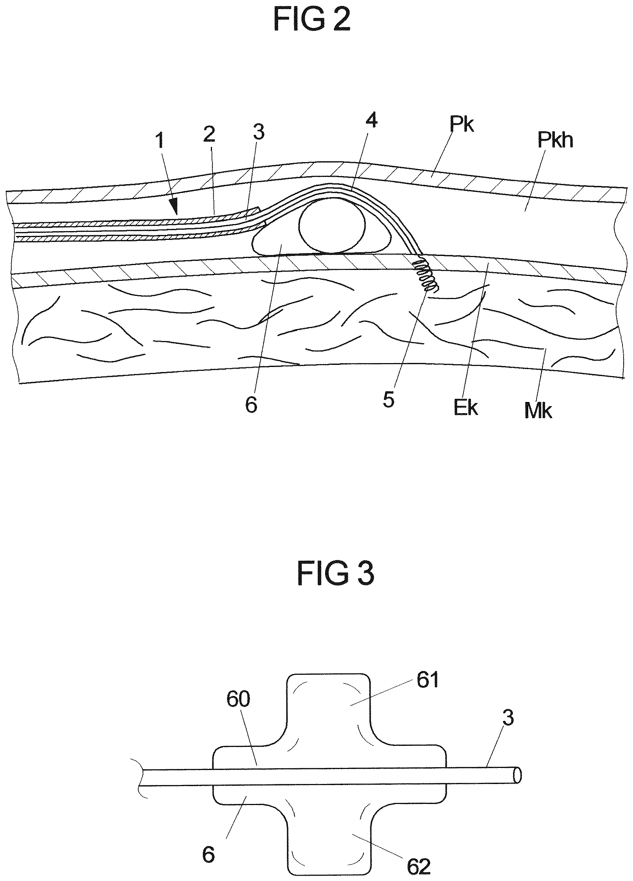

Fig. 2 zeigt in vergrößerter Darstellung den Bereich II gemäß Fig. 1 mit dem Perikard Pk, dem Epikard Ek und dem zwischen Perikard Pk und Epikard Ek gebildeten Perikardspalt Pkh sowie dem an das Epikard Ek anschließenden Myokard Mk des Herzens. Zur Implantation einer epikardialen Herzschrittmacherelektrode 4 wird ein Implantationskatheter 1 nach Punktion des Perikards Pk transkutan bzw. transthorakal in den Perikardspalt Pkh eingeführt und entlang des Perikardspaltes Pkh bewegt bis ein optimaler Implantationsort für die Implantation der epikardialen Herzschrittmacherelektrode 4 zur Herzmuskelerregung erreicht ist.

Der Implantationskatheter 1 umfasst einen schlauchförmigen, flexiblen Außenkatheter 2 und einen innerhalb des Außenkatheters 2 geführten schlauchförmigen, flexiblen Innenkatheter 3, in dem die epikardialen Herzschrittmacherelektrode 4 geführt ist, an deren distalem Ende ein vorzugsweise schrauben- oder korkenzieherförmig ausgebildeter Fixierungsmechanismus 5 angeordnet ist, der zur festen Verankerung im Epikard/Myokard des Herzens in das Epikard/Myokard eingeschraubt wird. Zu diesem Zweck wird in geeigneter Weise ein Drehmoment auf die Elektrode 4 ausgeübt, die derart ausgebildet ist, dass das Drehmoment auf das distale Ende der epikardiale Herzschrittmacherelektrode 4 übertragen wird.

Zur optimalen Verankerung der Elektrode 4 im Epikard/Myokard ist es wesentlich, dass die Elektrode 4 unter einem geeigneten Winkel in das Epikard/Myokard eingeschraubt wird. Darüber hinaus ist es für eine einwandfreie, reproduzierbare und sichere Implantation der Elektrode 4 wesentlich, dass die Lage der Elektrode 4 in Bezug auf das Perikard beim Einschrauben stabil ist, um einerseits den optimalen Implantationswinkel einzuhalten und andererseits den seitlichen Winkel senkrecht zu halten, so dass die Elektrode 4 in das Myokard geschraubt wird und nicht parallel zum Epikard, weil sie dann keinen Halt im Epikard/Myokard findet. Während des Einschraubens soll das auf die Elektrode 4 ausgeübte Drehmoment optimal auf die Elektrode 4 übertragen werden. Daher muss ein Einknicken des Innenkatheters 3 beim Einstellen des Implantationswinkels verhindert werden, andernfalls könnte die Reibung zwischen Innenkatheter 3 und Elektrode 4 so stark erhöht werden, dass ein Einschrauben der Elektrode 4 nicht mehr durchführbar ist. Dazu kann der Innenkatheter 3 nach Bedarf aus unterschiedlich flexiblen Materialien und/oder mit einem Drahtgeflecht ("Braiding") oder Drahtspirale ("Coiling") ausgestattet sein.

Zur Erfüllung der vorstehend genannten Aufgaben ist erfindungsgemäß ein formveränderliches Element vorgesehen, das in der nicht beanspruchten Ausführungsform gemäß Fig. 2 als Ballon 6 ausgebildet ist, der beim Einführen des Implantationskatheters 1 in den Perikardspalt Pkh innerhalb des Außenkatheters 2 angeordnet ist und am Implantationsort mit einem Gas oder einer Flüssigkeit befüllt wird, wodurch das Volumen des Ballons 6 vergrößert wird. Dadurch ändert die um den Ballon 6 geführte Elektrode 4 ihre Ausrichtung und nimmt einen vom Maß der Befüllung des Ballons 6 und damit dessen Volumenänderung abhängigen Implantationswinkel in Bezug auf das Epikard/Myokard ein.

Zur Lagestabilisierung des Ballons 6 und der um den Ballon 6 herum im Innenkatheter 3 geführten Elektrode 4 ist der Ballon 6 gemäß der in Fig. 3 dargestellten schematischen Draufsicht auf den kreuz- oder hammerförmigen Ballon 6 und den Innenkatheter 3 mit seitlich entfaltbaren Flügeln 61, 62 versehen, die die Anlagefläche des Ballons 6 innerhalb des Perikardspaltes vergrößern und damit ein Mitdrehen des Ballons 6 bzw. des mit dem Ballon 6 verbundenen oder entlang eines offenen oder geschlossenen Führungskanals 60 des Ballons 6 geführten Innenkatheters 3 beim Einschrauben der epikardialen Herzschrittmacherelektrode 5 verhindert wird. Insbesondere wird durch diese seitlichen Flügel dass sich die Elektrodenspitze beim Versuch des Einschraubens in das Epikard/Myokard parallel zur Oberfläche orientiert, wodurch es dann zu einem unerwünschten Abrutschen der Elektrodenspitze vom Epikard/Myokard kommen würde.

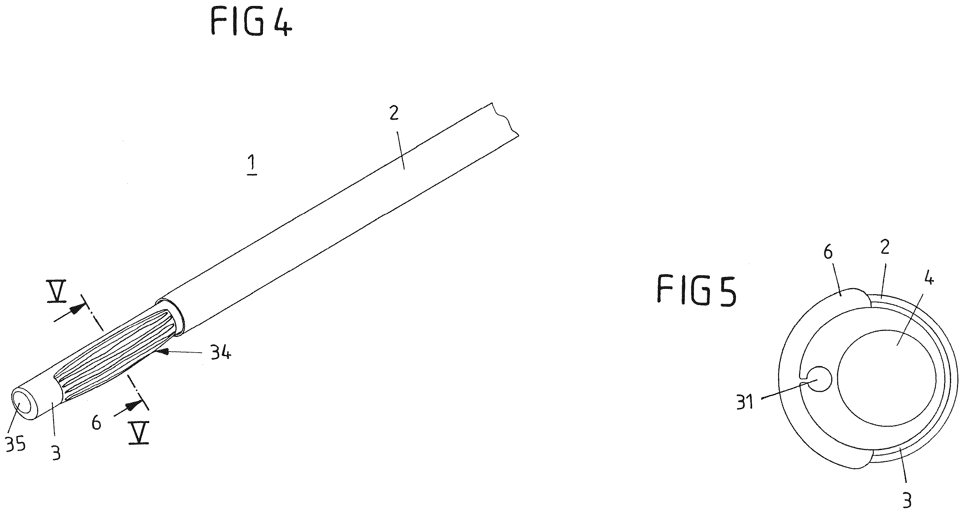

Fig. 4 zeigt in einer schematisch-perspektivischen Darstellung den Implantationskatheter 1 in der zum Einführen des Implantationskatheters 1 in den Perikardspalt Pkh vorgesehenen Form bis zum Erreichen des Implantationsortes und Fig. 5 einen Schnitt durch den Implantationskatheter 1 entlang der Linie V-V gemäß Fig. 4.

Der aus einem Außenkatheter 2 und einem im Lumen des Außenkatheters 2 geführten Innenkatheter 3 bestehende Implantationskatheter 1 weist am distalen Ende des Innenkatheters 3 die nicht befüllte Ballonhülle des Ballons 6 auf. Im Lumen des Innenkatheters 3 ist die Elektrode 4 in Längsrichtung geführt und eine Zufuhrleitung 31 zum Befüllen des Ballons 6 angeordnet oder vom Lumen zum Einführen der Elektrode 4 abgeteilt. Die Zufuhrleitung des Ballons 6 und die Elektrode 4 können auch in separaten Schläuchen geführt werden.

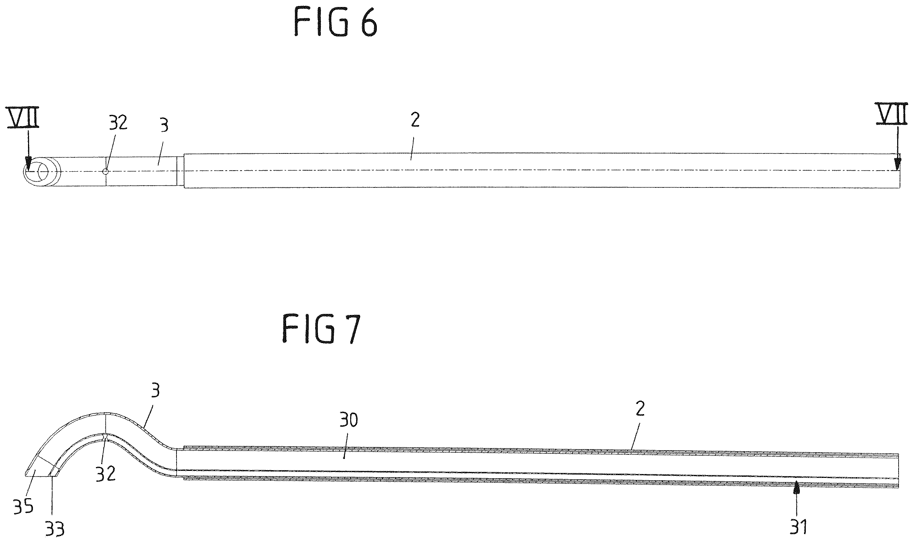

Fig. 6 zeigt in einer Draufsicht und Fig. 7 in einem Längsschnitt entlang der Linie VII-VII gemäß Fig. 6 den Implantationskatheter 1 mit dem Außenkatheter 2 und dem im Lumen des Außenkatheters 2 geführten Innenkatheter 3, der ein Lumen 30 zum Einführen der Elektrode 4 und ein als Zufuhrleitung 31 dienendes Lumen zum Befüllen des in den Fig. 6 und 7 nicht dargestellten Ballons 6 aufweist. Im proximalen Endbereich des Innenkatheters 3 ist eine Öffnung 32 zum Befüllen des Ballons 6 vorgesehen, während das distale Ende der Zufuhrleitung 31 zum Befüllen des Ballons 6 durch eine Wand 33 verschlossen ist. Das distale Ende des Lumens 30 zum Einführen der Elektrode 4 weist eine Öffnung 35 auf, durch die die Elektrode 4 geführt wird. Zusätzlich dient die distale Öffnung 35 dazu, am Implantationsort den Innenkatheter 3 an das Epikard anzusaugen, wozu am proximalen Ende des Lumens 30 zum Einführen der Elektrode 4 ein Gerät zur Erzeugung eines Unterdrucks angeschlossen wird.

Alternativ zu einem innerhalb des Innenkatheters 3 geführten Lumen 31 zum Befüllen des Ballons 6 kann eine Befüllleitung an der Außenseite des Außenkatheters 2 entlang zum Ballon 6 geführt werden oder außerhalb des Innenkatheters 3 im Lumen des Außenkatheters 2 angeordnet werden. Als weitere Alternative sind zwei getrennte Schläuche als Innenkatheter 3 möglich, von denen der eine als Ballonzuleitung und der andere als Elektrodenführung dient.

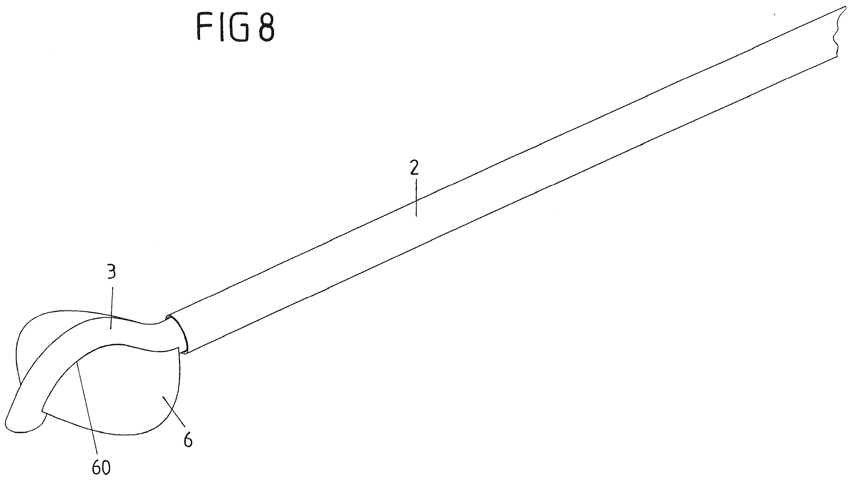

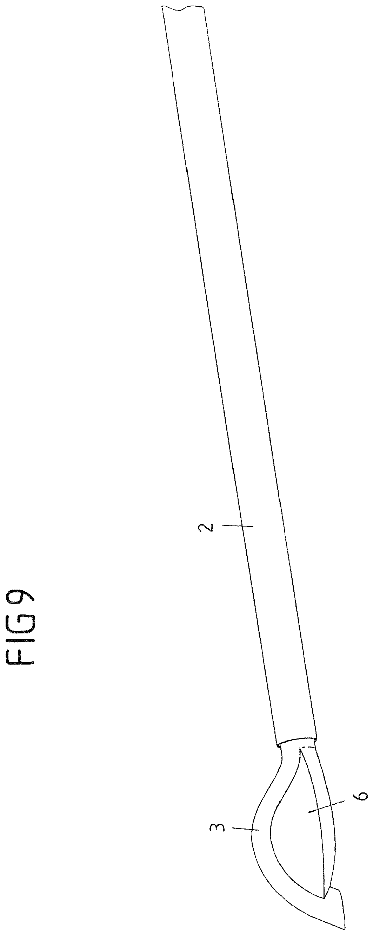

Fig. 7 zeigt am distalen Ende des Innenkatheters 3 eine Wölbung, die durch das Befüllen des Ballons 6 gemäß den Fig. 8 und 9 hervorgerufen wird, wobei das Befüllen des Ballons 6 sowohl mit einem Gas als auch mit einer Flüssigkeit erfolgen kann, die über das Lumen 31 und die Öffnung 32 dem Ballon 6 zugeführt werden.

Fig. 8 und 9 zeigen schematisch in perspektivischer Darstellung den am Implantationsort befüllten Ballon 6 und verdeutlicht die durch das Befüllen des Ballons 6 erzielte Ausrichtung des Innenkatheters 3 in einem vom Befüllungsgrad des Ballons 6 abhängigen Implantationswinkel, unter dem die Elektrode 4 in das Epikard/Myokard eingeschraubt wird. Zur Stabilisierung der Lage des Innenkatheters 3 mit der darin geführten Elektrodenleitung 4 ist im oder am Ballon 6 ein Führungskanal 60 vorgesehen, in dem der Innenkatheter 3 gelagert ist bzw. über den der Innenkatheter 3 mit dem Ballon 6 verbunden ist, so dass beim Einschrauben der epikardialen Herzschrittmacherelektrode 4 in das Epikard/Myokard eine Lagerveränderung des Innenkatheters 3 und damit des Implantationswinkels vermieden wird.

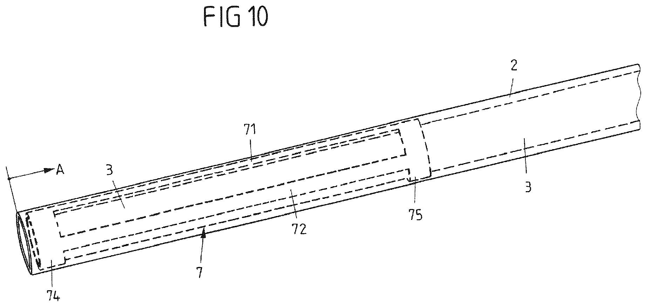

In den Fig. 10 bis 12 ist ein zweites Ausführungsbeispiel für ein formveränderliches Element zur Ausrichtung bzw. Einstellung des Implantationswinkels und zur Stabilisierung eines Implantationskatheters zum Implantieren einer epikardialen Herzschrittmacherelektrode dargestellt.

Die zweite Ausführungsform eines formveränderlichen Elements besteht gemäß Fig. 10 aus einem im Transport- oder Ausgangszustand, d.h. heißt vor dem Erreichen des Implantationsortes, in das distale Ende des Außenkatheters 2 eingezogenen, gestrichelt dargestellten Ausricht- und Stabilisationselement 7 mit endseitigen offenen oder geschlossenen Ringen 74, 75, die über in Längsrichtung verlaufende Streben 71 bis 73 verbunden sind. Im Innern des im Außenkatheter 2 geführten Ausricht- und Stabilisationselements 7 ist das distale Ende des Innenkatheters 3 geführt, in dem die Elektrode 4 angeordnet ist. Die die endseitigen Ringe 74, 75 verbindenden Streben 71 bis 73 bestehen aus einer mittleren Ausrichtungsstrebe 71 zum Einstellen des Implantationswinkels und zwei seitlichen Stützstreben 72, 73 zur Lagestabilisierung.

Alternativ zu der in Fig. 10 dargestellten Zylinderform des Ausricht- und Stabilisationselement 7 sind auch andere Formen möglich. Insbesondere muss das Ausricht- und Stabilisationselement 7 nicht rundum geschlossen sein, um ein Schlitzen des Implantationskatheters nach erfolgreicher Implantation zu ermöglichen. Auch müssen die Streben 71 - 73 nicht als gerade Stäbe ausgeführt werden, sondern können auch gekrümmt sein, was unter Umständen auch erforderlich ist. Auch die Anzahlen der Ausrichtungsstreben 71 und seitlichen Stützstreben 72, 73 ist variabel.

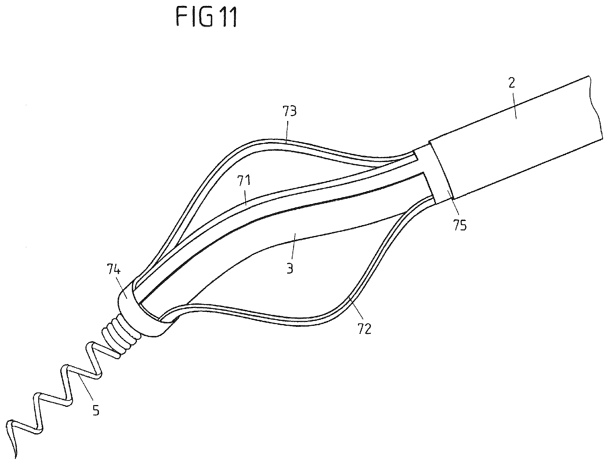

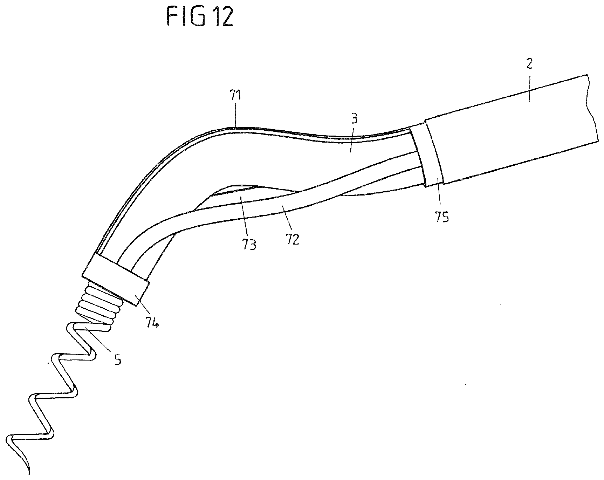

In den Fig. 11 und 12 ist die Form des Ausricht- und Stabilisationselements 7 am Implantationsort in zwei unterschiedlichen perspektivischen Ansichten dargestellt, in denen aus der in Fig. 10 dargestellten Zylinderform des Ausricht- und Stabilisationselements 7 beispielsweise durch Zurückziehen des Außenkatheters 2 in Richtung des Pfeiles A gemäß Fig. 10 die Streben 71 bis 73 eine Form annehmen, die den von den endseitigen Ringen 74, 75 eingefassten Innenkatheter 3 in die gewünschte Ausrichtung zur Einstellung eines vorgegebenen Implantationswinkels bringen und gleichzeitig die Lage des distalen Endes des Innenkatheters 3 am Implantationsort stabilisieren.

Die Formveränderung des Ausricht- und Stabilisationselements 7 von der in Fig. 10 dargestellten Zylinderform in die in den Fig. 11 und 12 dargestellte Ausrichtungs- und Stabilisierungsform kann auf unterschiedliche Weise realisiert werden. So können die Streben 71 bis 73 aus einem Material mit elastischer Rückstellkraft beispielsweise in Form von Federn oder Federstahl eingesetzt werden, die die in Fig. 10 dargestellte gestreckte Form einnehmen, solange sie vom Außenkatheter 2 ganz oder teilweise umfasst sind. Auch Kunststoffe sind denkbar. Erreicht der in den Perikardspalt Pk eingeführte Implantationskatheter 1 den Implantationsort, so wird durch Zurückziehen des Außenkatheters 2 in Richtung des proximalen Endes des Implantationskatheters 1 die Zwangsform der Streben 71 bis 73 aufgehoben und die Streben 71 bis 73 nehmen eine durch die elastische Rückstellkraft vorgegebene Form entsprechend den Fig. 11 und 12 ein.

Alternativ kann die Pseudoelastizität eines Formgedächtnismaterials bzw. einer Formgedächtnislegierung genutzt werden, bei der der in den Fig. 11 und 12 dargestellte gekrümmte Zustand die selbständig eingenommene Form ist. Durch Hineinziehen des Käfigs 7 in den Außenkatheter 2 wird das Ausricht- und Stabilisationselement 7 in die gestreckte, in Fig. 10 dargestellte Form gebracht. Am Implantationsort wird zur Ausrichtung des Innenkatheters 3 und damit zur Einstellung des Implantationswinkels der Außenkatheter 2 in Richtung des Pfeils A gemäß Fig. 10 zurückgezogen, so dass das Ausricht- und Stabilisationselement 7 die gekrümmte Form wieder selbständig einnimmt.

Als Formgedächtnismaterial für das Ausricht- und Stabilisationselement 7 kann beispielsweise die Nickel-Titan-Legierung Nitinol eingesetzt werden, deren temperaturabhängiger Formgedächtniseffekt in einer alternativen Ausführungsform des Ausricht- und Stabilisationselements 7 genutzt werden kann. Dabei beeinflusst das Legierungsverhältnis die Transformationstemperatur, wobei vorzugsweise ein aus einer Legierung mit einer tiefen Transformationstemperatur von zum Beispiel 0°C bestehendes Formgedächtnismaterial verwendet wird, das sich bei Raum- oder Körpertemperatur ähnlich wie Federstahl verhält und damit am Implantationsort die gewünschte gekrümmte Form einnimmt. Alternativ können auch andere Legierungen oder Kunststoffe mit Formgedächtniseffekt eingesetzt werden.

Neben Materialien mit einem temperaturabhängigen Formgedächtniseffekt können auch Materialien mit durch andere Effekte ausgelöster Formveränderung, beispielsweise durch Einwirken eines äußeren Magnetfeldes beeinflussbare Materialien eingesetzt werden.

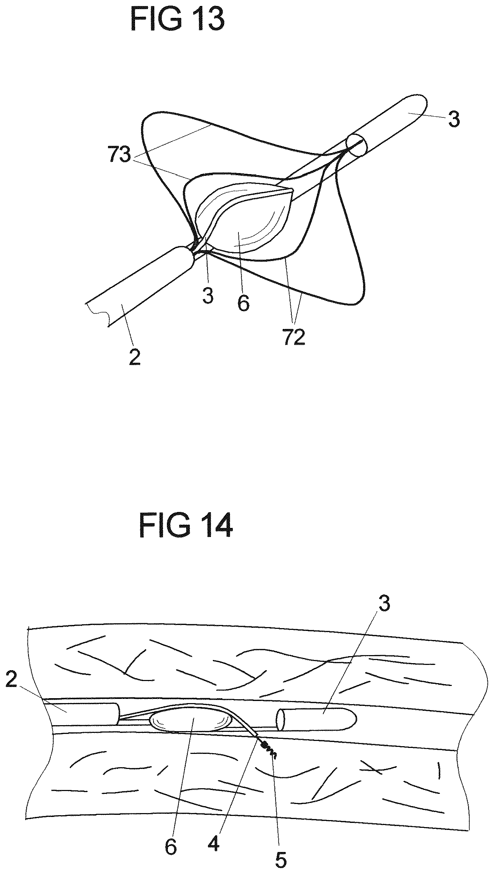

In den Fig. 13 und 14 ist in einer perspektivischen Draufsicht und in einer schematischen Seitenansicht eine weitere Ausführungsform für ein formveränderliches Element dargestellt, das sich aus Teilen der vorstehend anhand der Fig. 2 bis 9 und 10 bis 11 beschriebenen Ausführungsbeispiele für ein formveränderliches Element zusammensetzt.

Diese weitere Ausführungsform eines formveränderlichen Elements besteht aus einem Ballon 6 zur Ausrichtung bzw. Einstellung des Implantationswinkels, mit dem die epikardiale Herzschrittmacherelektrode 4 in das Epikard/Myokard eingeschraubt wird, und beispielsweise aus Nitinol bestehenden Stützstreben 72, 73 zur Lagestabilisierung. Im Außenkatheter 2 werden z.B. zwei Innenkatheter (Innenkatheter und Elektrodenführung) geführt. Der Au ßenkatheter 2 wird am Implantationsort zurückgezogen und setzt dadurch die Nitinolstreben und den Ballon 6 frei. Die Nitinolstreben, befestigt am Innenkatheter 3, stabilisieren daraufhin die seitliche Lage des Innenkatheters 3 und verhindern ein Wegkippen. Das Volumen des Ballons 6 wird durch Befüllen vergrößert bis die im Lumen des Innenkatheters 3 geführte Elektrodenführung und damit die darin geführte Elektrode 4 den gewünschten Implantationswinkel eingenommen hat. Durch Zurückziehen des Außenkatheters 2 gegenüber dem Innenkatheter 3 kann vorher, gleichzeitig oder anschließend das Ausricht- und Stabilisationselement 7 mit den Streben 72, 73 freigegeben werden, so dass diese aus der gestreckten Form unter Wirkung des Formgedächtniseffektes in die gekrümmte Form gemäß Fig. 13 übergehen und damit die Lage des distalen Endes des Implantationskatheters 1 stabilisieren.

Nach erfolgter Implantation der epikardialen Herzschrittmacherelektrode 5 muss der Implantationskatheter mit dem formveränderlichen Element 6, 7 zurückgezogen werden, was aber dadurch verhindert wird, dass am proximalen Ende der Elektrodenleitung ein Stecker zur Verbindung der Elektrodenleitung mit einem Herzschrittmacher angeordnet ist. Dieser Stecker weist üblicherweise einen größeren Durchmesser als der Implantationskatheter 1 auf, so dass nach erfolgter Implantation der Implantationskatheter 1 nicht mehr ohne weiteres entfernt werden kann. Aus diesem Grunde werden die Katheter mit einem speziellen Messer während des Herausziehens der Länge nach aufgeschnitten und können so am Stecker vorbei bewegt werden.

Zu diesem Zweck kann die Möglichkeit des Aufschlitzens in den Implantationskatheter integriert werden, wobei beispielsweise die endseitigen Ringe 74, 75 des Ausricht- und Stabilisationselements 7 nicht geschlossen, sondern auf einer Seite offen sind, um das Ausricht- und Stabilisationselement 7 an dem speziellen Messer vorbeizuführen.

Da die Implantation der epikardialen Herzschrittmacherelektrodenspitze (Helix) 5 nicht durch das Gefäßsystem zum Herzen, sondern transkutan bzw. transthorakal erfolgt, kann alternativ der Implantationskatheter 1 einen größeren Durchmesser aufweisen, so dass bei einem entsprechenden Durchmesser des proximalen Steckers, der kleiner ist als der Innendurchmesser des Katheters, der Implantationskatheter, d.h. der Außenkatheter bzw. die Kombination aus Außenkatheter und Innenkatheter, von der Elektrodenleitung abgezogen werden kann, ohne dass die Katheter der Länge nach aufgeschlitzt werden müssen.

### Bezugszeichenliste

- 1: Implantationskatheter
- 2: Außenkatheter
- 3: Innenkatheter
- 4: Epikardiale Herzschrittmacherelektrode
- 5: Distaler Fixierungsmechanismus (Helix)
- 6: Ballon
- 7: Ausricht- und Stabilisationselement
- 30: Lumen
- 31: Gas- oder Flüssigkeitsleitung
- 32: Öffnung
- 33: Wand
- 34: fensterförmiger Ausschnitt
- 35: Öffnung
- 60: Führungskanal
- 61, 62: Lagestabilisatoren (entfaltbare Ballonflügel)
- 71: Ausrichtungsstrebe
- 72, 73: Lagestabilisatoren (Stützstreben)
- 74, 75: endseitige Ringe
- Ek: Epikard
- Mk: Myokard
- Pk: Perikard
- Pkh: Perikardspalt

## Patentansprüche

1. Vorrichtung zur transkutanen Implantation einer epikardialen Herzschrittmacherelektrode, mit einem schlauchförmigen, flexiblen und in den Perikardspalt einführbaren Implantationskatheter und einer epikardialen Herzschrittmacherelektrode, die in dem Implantationskatheter angeordnet ist, wobei der distale Endbereich des Implantationskatheters (1) ein formveränderliches Element (6, 7) zur Einstellung des Implantationswinkels und zur Stabilisierung, insbesondere zur seitlichen Stabilisierung, der Elektrode (4) aufweist,
**dadurch gekennzeichnet,**
**dass** der Implantationskatheter (1) einen Außenkatheter (2) umfasst und dass das formveränderliche Element aus einem die Elektrode (4) aufnehmenden Ausricht- und Stabilisationselement (7) mit endseitigen offenen oder geschlossenen Ringen (74, 75) und die endseitigen Ringe (74, 75) verbindenden Streben (71-73) besteht, wobei die Streben (71-73) beim Vorschub
des Implantationskatheters (1) von der Punktionsstelle zum Implantationsort im Wesentlichen parallel zur Wand des Außenkatheters (2) ausgerichtet sind und sich nach Freisetzen des Ausricht- und Stabilisationselements (7) aus dem Außenkatheter (2) zu einer Implantationsposition entfalten.

2. Vorrichtung nach Anspruch 1, **dadurch gekennzeichnet, dass** in der Implantationsposition mindestens eine mittlere Ausrichtungsstrebe (71) die Elektrode (4) ausrichtet und mindestens zwei Stützstreben (72, 73) die Ausrichtung und Lage der Elektrode (4) stabilisieren.

3. Vorrichtung nach Anspruch 1 oder 2, **dadurch gekennzeichnet, dass** zumindest die die endseitigen Ringe (74, 75) verbindenden Streben (71-73) des Ausricht- und Stabilisationselements (7) aus einem Formgedächtnismaterial, insbesondere aus Nitinol, einem Federmaterial, insbesondere Federstahl oder Kunststoff, oder einem pseudoelastischen Material, insbesondere aus Nitinol, bestehen.

4. Vorrichtung nach Anspruch 1 oder 2, **dadurch gekennzeichnet, dass** zumindest die die endseitigen Ringe (74, 75) verbindenden Streben (71-73) des Ausricht- und Stabilisationselements (7) aus einem Material mit elastischer Rückstellkraft bestehen.

5. Vorrichtung nach mindestens einem der vorangehenden Ansprüche, **dadurch gekennzeichnet, dass** die Elektrode (4) in einem schlauchförmigen, flexiblen, innerhalb eines Außenkatheters (2) geführten Innenkatheter (3) des Implantationskatheters (1) angeordnet ist.

6. Vorrichtung nach Anspruch 5, **dadurch gekennzeichnet, dass** das Ausricht- und Stabilisationselement (7) zwischen dem Innenkatheter (3) und dem Außenkatheter (2) angeordnet und mit dem distalen Ende des Innenkatheters (3) verbunden ist.

7. Vorrichtung nach Anspruch 5 oder 6, **dadurch gekennzeichnet, dass** das proximale Ende des Implantationskatheters (1) mit einer Vorrichtung zum Ansaugen des distalen Endes des Implantationskatheters (1) an das Epikard (Ek) verbunden ist.

8. Vorrichtung nach mindestens einem der Ansprüche 5 bis 7, **dadurch gekennzeichnet, dass** das proximale Ende des Innenkatheters (3) und/oder des Außenkatheters (2) mit einer Einrichtung zum längsseitigen Aufschlitzen des Innenkatheters (3) und/oder des Außenkatheters (2) beim Zurückziehen des Innenkatheters (3) und/oder des Außenkatheters (2) verbunden ist.

9. Vorrichtung nach mindestens einem der vorangehenden Ansprüche, **dadurch gekennzeichnet, dass** das am distalen Ende des Implantationskatheters (1) angeordnete formveränderliche Element (6, 7) aus einem aufblasbaren, über eine Zufuhrleitung (31) mit dem proximalen Ende des Implantationskatheters (1) verbundenen Ballon (6) und einem Ausricht- und Stabilisationselement (7) mit endseitigen Ringen (74, 75) und die endseitigen Ringe (74, 75) verbindenden flexiblen Streben (71-73) aus einem Formgedächtnismaterial, insbesondere aus Nitinol, besteht.

## Claims

1. Device for the transcutaneous implantation of an epicardial pacemaker electrode, with a tubular, flexible implantation catheter insertable into the pericardial space and with an epicardial pacemaker electrode, which is arranged in the implantation catheter, wherein the distal end area of the implantation catheter (1) has a shape-variable element (6, 7) for adjusting the implantation angle and for stabilizing, in particular laterally stabilizing, the electrode (4),
**characterized in that**
the implantation catheter (1) comprises an outer catheter (2) and **in that** the shape-variable element is composed of an aligning and stabilizing element (7), which receives the electrode (4) and which has open or closed end-side rings (74, 75) and struts (71-73) connecting the end-side rings (74, 75), wherein during the advance of the implantation catheter (1) from the puncture site to the implantation site the struts (71-73) are oriented substantially parallel to the wall of the outer catheter (2) and, after release of the aligning and stabilizing element (7), deploy out of the outer catheter (2) to an implantation position.

2. Device according to Claim 1, **characterized in that,** in the implantation position, at least one central aligning strut (71) aligns the electrode (4), and at least two support struts (72, 73) stabilize the alignment and position of the electrode (4).

3. Device according to Claim 1 or 2, **characterized in that** at least the struts (71-73) of the aligning and stabilizing element (7) that connect the end-side rings (74, 75) are made from a shape-memory material, in particular from nitinol, a spring material, in particular spring steel or plastic, or a pseudoelastic material, in particular from nitinol.

4. Device according to Claim 1 or 2, **characterized in that** at least the struts (71-73) of the aligning and stabilizing element (7) that connect the end-side rings (74, 75) are made from a material with elastic restoring force.

5. Device according to at least one of the preceding claims, **characterized in that** the electrode (4) is arranged in a tubular, flexible inner catheter (3) of the implantation catheter (1) guided inside the outer catheter (2).

6. Device according to Claim 5, **characterized in that** the aligning and stabilizing element (7) is arranged between the inner catheter (3) and the outer catheter (2) and is connected to the distal end of the inner catheter (3).

7. Device according to Claim 5 or 6, **characterized in that** the proximal end of the implantation catheter (1) is connected to a device for sucking the distal end of the implantation catheter (1) onto the epicardium (Ek).

8. Device according to one of Claims 5 to 7, **characterized in that** the proximal end of the inner catheter (3) and/or outer catheter (2) is connected to a mechanism for longitudinally slitting open the inner catheter (3) and/or outer catheter (2) during the withdrawal of the inner catheter (3) and/or outer catheter (2).

9. Device according to at least one of the preceding claims, **characterized in that** the shape-variable element (6, 7) arranged at the distal end of the implantation catheter (1) is composed of an inflatable balloon (6), which is connected via a supply line (31) to the proximal end of the implantation catheter (1), and of an aligning and stabilizing element (7) with end-side rings (74, 75) and with flexible struts (71-73) which connect the end-side rings (74, 75) and are made from a shape-memory material, in particular from nitinol.

## Revendications

1. Dispositif d'implantation transcutanée d'une électrode épicardique de stimulateur cardiaque, ledit dispositif comprenant un cathéter d'implantation tubulaire, flexible et insérable dans l'espace péricardique et une électrode épicardique de stimulateur cardiaque qui est disposée dans le cathéter d'implantation, la zone d'extrémité distale du cathéter d'implantation (1) comportant un élément à changement de forme (6, 7) destiné à régler l'angle d'implantation et à stabiliser, en particulier à stabiliser latéralement, l'électrode (4),
**caractérisé en ce que**
le cathéter d'implantation (1) comprend un cathéter extérieur (2) et l'élément à changement de forme comprend un élément d'orientation et de stabilisation (7) qui reçoit l'électrode (4) et qui est pourvu d'anneaux (74, 75) ouverts ou fermés aux extrémités et d'entretoises (71-73) reliant les anneaux (74, 75), les entretoises (71-73) étant orientées sensiblement parallèlement à la paroi du cathéter extérieur (2) lorsque le cathéter d'implantation (1) est avancé du point de ponction au site d'implantation et se déployant vers une position d'implantation après que l'élément d'orientation et de stabilisation (7) a été libéré du cathéter extérieur (2).

2. Dispositif selon la revendication 1, **caractérisé en ce que**, dans la position d'implantation, au moins une entretoise d'orientation centrale (71) oriente l'électrode (4) et au moins deux entretoises d'appui (72, 73) stabilisent l'orientation et la position de l'électrode (4).

3. Dispositif selon la revendication 1 ou 2, **caractérisé en ce qu'**au moins les entretoises (71-73) qui relient les anneaux d'extrémité (74, 75) de l'élément d'orientation et de stabilisation (7) sont en une matière à mémoire de forme, notamment en nitinol, en matière à ressort, notamment en acier à ressort ou en matière synthétique, ou en matière pseudo-élastique, notamment en nitinol.

4. Dispositif selon la revendication 1 ou 2, **caractérisé en ce qu'**au moins les entretoises (71-73), reliant les anneaux d'extrémité (74, 75), de l'élément d'orientation et de stabilisation (7) sont en une matière à force de rappel élastique.

5. Dispositif selon l'une au moins des revendications précédentes, **caractérisé en ce que** l'électrode (4) est disposée dans un cathéter intérieur (3), tubulaire, flexible et guidé à l'intérieur d'un cathéter extérieur (2), du cathéter d'implantation (1).

6. Dispositif selon la revendication 5, **caractérisé en ce que** l'élément d'orientation et de stabilisation (7) est disposé entre le cathéter intérieur (3) et le cathéter extérieur (2) et est relié à l'extrémité distale du cathéter intérieur (3).

7. Dispositif selon la revendication 5 ou 6, **caractérisé en ce que** l'extrémité proximale du cathéter d'implantation (1) est reliée à un dispositif destiné à aspirer l'extrémité distale du cathéter d'implantation (1) sur l'épicarde (Ek) .

8. Dispositif selon l'une au moins des revendications 5 à 7, **caractérisé en ce que** l'extrémité proximale du cathéter intérieur (3) et/ou du cathéter extérieur (2) est reliée à un moyen destiné à fendre longitudinalement le cathéter intérieur (3) et/ou le cathéter extérieur (2) pendant que le cathéter intérieur (3) et/ou le cathéter extérieur (2) est retiré.

9. Dispositif selon l'une au moins des revendications précédentes, **caractérisé en ce que** l'élément à changement de forme (6, 7), disposé à l'extrémité distale du cathéter d'implantation (1), comprend un ballonnet gonflable (6), qui est relié à l'extrémité proximale du cathéter d'implantation (1) par un conduit d'alimentation (31), et un élément d'orientation et de stabilisation (7) qui est pourvu d'anneaux d'extrémité (74, 75) et d'entretoises flexibles (71-73) en matière à mémoire de forme, notamment en Nitinol, qui relient les anneaux d'extrémité (74, 75).
